# FASCICULE DE BREVET EUROPEEN

(11) **EP 1 879 655 B1**
(45) Date de publication et mention de la délivrance du brevet: **26.11.2014**
(21) Numéro de dépôt: 06744753.2
(22) Date de dépôt: 15.05.2006
(51) Int. Cl.: A61P 31/04, A61K 45/06, A61K 31/045, A61K 31/05, A61K 31/43

(54) **Combinaison pharmaceutique comprenant un antibactérien et une substance active choisie parmi le carvéol, le carvacrol, l'alpha-ionone, le beta-ionone, et le thymol**
Pharmazeutische Zusammensetzung mit einem antibakteriellen Mittel und einem aus Carveol, Carvacrol, Alpha-Ionone, Beta-Ionone und Thymol ausgewählten Wirkstoff
Pharmaceutical composition comprising an anti-bacterial agent and an active ingredient selected from carveol, carvacrol, alpha-ionone, beta-ionone, and thymol

(30) Priorité: 13.05.2005 WO PCT/IB2005/001313
(43) Date de publication de la demande: 23.01.2008
(73) Titulaire: Advanced Scientific Developments, 20200 Casablanca (MA)
(72) Inventeur: REMMAL, Adnane, 30000 Fes (MA)
(74) Mandataire: Gallois, Valérie
(86) Numéro de dépôt international: PCT/IB2006/001350
(87) Numéro de publication internationale: WO 2006/120567

(56) Documents cités:
- EP-A- 0 264 660
- WO-A-00/10390
- WO-A-00/69277
- WO-A-02/41899
- WO-A-96/40192
- WO-A-2004/052308
- WO-A-2004/070017
- WO-A-2005/113128
- US-A1- 2003 064 948
- FANG J-Y ET AL: "Efficacy and irritancy of enhancers on the in-vitro and in-vivo percutaneous absorption of curcumin" JOURNAL OF PHARMACY AND PHARMACOLOGY 01 MAY 2003 UNITED KINGDOM, vol. 55, no. 5, 1 mai 2003 (2003-05-01), pages 593-601, XP008057082 ISSN: 0022-3573
- TANAKA, YASUO ET AL: "Antibacterial activity of essential oils and oleoresins of spices and herbs against pathogens bacteria in upper airway respiratory tract" NIPPON SHOKUHIN KAGAKU GAKKAISHI (2002), 9(2), 67-76 CODEN: NSKGF4; ISSN: 1341-2094, 2002, XP008037275

## Description

L'invention concerne une composition pharmaceutique comprenant deux substances thérapeutiquement actives dont l'une exerce une action de potentialisation sur l'autre, ainsi que l'utilisation de cette composition.

Il est connu que l'efficacité des agents thérapeutiques dépend des doses utilisées, ce qui oblige, dans le cas des résistances partielles à augmenter les doses des agents thérapeutiques pour atteindre l'efficacité recherchée. Cette augmentation de la dose conduit à des problèmes d'apparition d'effets secondaires indésirables et de toxicité aiguë ou chronique, pouvant compliquer considérablement l'état des patients traités.

Cette résistance partielle peut devenir une résistance totale. Dans ce cas, l'augmentation des doses n'a plus aucun effet thérapeutique bénéfique, seuls les effets de toxicité sont observés. Le traitement consiste alors à changer l'agent thérapeutique.

Cette cascade peut se répéter et conduire à la situation la plus grave: la résistance totale à de multiples agents thérapeutiques (multi-drug résistance).

Ainsi, en particulier, les malades Immunodéprimés, deviennent de plus en plus difficiles à traiter et leur espérance de vie en est réduite d'autant. De plus, leur confort de vie est largement affecté par l'administration à hautes doses d'agents thérapeutiques.

L'invention a pour but de pallier ces problèmes en proposant d'associer au moins deux substances thérapeutiquement actives, dont l'une potentialise l'activité de l'autre, ce qui permet non seulement d'abaisser les doses de chaque substance thérapeutiquement active mais également de traiter les patients atteints d'infections à germes résistants.

A cet effet, l'invention propose une composition pharmaceutique caractérisée en ce qu'elle comprend:
- au moins une première substance thérapeutiquement active, et
- au moins une seconde substance thérapeutiquement active qui est un antibiotique, dans laquelle les premières et secondes substances sont choisies comme défini dans la revendication 1.

La première substance thérapeutique peut être obtenue simplement par synthèse chimique ou à partir d'une source végétale. Des antibiotiques connus sont les bêtalactamines, les céphalosphorines, la fosfomycine, les glycopeptides, les polymyxines, les gramicidines, la tyrocidine, les aminosides, les macrolides, les lincosamides, les synergistines, les phénicolés, les tétracyclines, l'acide fusidique, les oxazolidinones, les rifamycines, les quinolones, les fluoroquinolones, les produits nitrés, les sulfamides, la triméthoprime, et leurs mélanges; ou encore

les pénicillines, l'oxacilline, la cloxaciline, l'ampicilline, l'amoxicilline, la bacampicilline, la métampicilline, la pivampicilline, l'azlocilline, la mezlocilline, la pipéracilline, la ticarcilline, le pivmécillinam, le sulbactam, le tazobactam, l'imipénème, la céfalexine, le céfadroxil, le céfaclor, la céfatrizine, la céfalotine, la céfapyrine, la céfazoline, la céfoxitine, le céfamandole, le céfotétan, le céfuroxime, le céfotaxime, le céfsulodine, le céfopérazone, le céfotiam, la céftazidime, le céftriaxone, le céfixime, le céfpodoxime, le céfépime, le latamoxef, l'aztréonam; la vancomycine, la vancocine, la téicoplanine, la polymyxine B, la colistine, la bacitracine, la tyrothricine, la streptomycine, la kanamycine, la tobramycine, l'amikacine, la sisomicine, la dibékacine, la nétilmicine, la spectinomycine, la spiramycine, l'érythromycine, la josamycine, la roxithromycine, la clarithromycine, l'azithromycine, la lincomycine, la clindamycine, la virginiamycine, la pristinamycine, la dalfopristine-quinupristine, le chloramphénicol, le thiamphénicol, la tétracycline, la doxycycline, la minocycline, l'acide fusidique, la linézolide, la rifamycine, la rifampicine, l'acide nalidixique, l'acide oxolinique, l'acide pipémidique, la fluméquine, la péfloxacine, la norfloxacine, l'ofloxacine, la ciprofloxacine, l'enoxacine, la sparfloxacine, la lévofloxacine, la moxifloxacine, la nitroxoline, le tilboquinol, la nitrofurantoïne, la nifuroxazide, la métronidazole, l'omidazole, la sulfadiazine, le sulfaméthisol, le triméthoprime, l'isoniazide et leurs dérivés et mélanges. Ces antibiotiques, et plus particulièrement l'amoxicilline, peuvent éventuellement être utilisés en combinaison avec l'acide clavulanique.

Une composition antibiotique tout particulièrement préférée décrite dans la revendication 1 est une composition dans laquelle la première substance thérapeutiquement active est le carvacrol et l'antibiotique est l'amoxicilline ou la rifampicine.

Une autre composition antibiotique tout particulièrement préférée décrite dans la revendication 1 est une composition antibiotique dans laquelle ladite première substance thérapeutiquement active est le carvéol et l'antibiotique est l'ampicilllne, le chloramphénicol, la tétracycline, la streptomycine, l'érythromycine, ou la polymixine B.

Encore une autre composition antibiotique tout particulièrement préférée décrite dans la revendication 1 est une composition antibiotique dans laquelle ladite première substance thérapeutiquement active est l'alpha-ionone ou la bêta-ionone et l'antibiotique est la céphazoline.

Toujours une autre composition antibiotique tout particulièrement préférée décrite dans la revendication 1 est une composition antibiotique dans laquelle ladite première substance thérapeutiquement active est le thymol et l'antibiotique est l'isoniazide.

Egalement, une composition antibiotique dans laquelle ladite première substance thérapeutiquement active est le carvacrol et l'antibiotique est l'amoxicilline associée à l'acide clavulanique est tout particulièrement préférée, et est décrite dans la revendication 1.

L'invention propose également une trousse (kit) caractérisée en ce qu'elle contient au moins un premier récipient contenant une première substance thérapeutiquement active, et au moins un second récipient contenant une seconde substance thérapeutiquement active qui est un antibiotique, dans laquelle les premières et secondes substances sont choisies comme défini dans la revendication 1..

L'invention propose enfin un traitement d'une infection due à une bactérie caractérisée en ce qu'on administre à un patient atteint d'une infection due à une bactérie, de manière simultanée ou séquentielle au moins une première substance thérapeutiquement active, et au moins une seconde substance thérapeutiquement active qui est un antibiotique, dans laquelle les premières et secondes substances sont choisies comme défini dans la revendication 1.

De préférence, on administre de manière simultanée ou séquentielle, à un patient atteint d'une infection due à une bactérie entre 10 et 200 mg/kg de poids du patient/jour de ladite première substance thérapeutiquement active, et entre 1 et 100 mg/kg de poids du patient/jour de ladite seconde substance thérapeutiquement active qui est un antibiotique.

L'invention sera mieux comprise et d'autres buts et avantages de celle-ci apparaîtront plus clairement à la lecture de la description explicative qui suit et qui est faite en référence à la figure unique annexée qui représente les résultats du test *in vivo* réalisés sur des souris expérimentalement infectées avec *Klebsiella Pneumonia* à forte résistance, et soit non traitées, soit traitées avec de l'amoxicilline seule, soit traitées avec du carvéol seul, soit traitées avec une composition pharmaceutique antibactérienne selon l'invention contenant de l'amoxicilline et du carvéol.

La composition pharmaceutique de l'invention comprend en tant que première substance thérapeutiquement active le thymol, le carvacrol, le carvéol, l'alpha-ionone, la bêta-ionone, leurs isomères ainsi que leurs mélanges, sous forme pure.

Ces composés ont des propriétés antibiotiques bien connues en eux-mêmes.

Le thymol, le carvacrol et le carvéol, l'alpha-ionone, la bêta-ionone se trouvent en proportions variées dans différents extraits de plantes aromatiques, c'est-à-dire qu'ils peuvent être purifiés à partir de ces plantes. Cependant, ils peuvent être tout simplement obtenus par synthèse chimique.

Or, les inventeurs ont maintenant découvert que ces composés ont un effet de potentialisation sur de nombreuses substances thérapeutiquement actives dont des antibiotiques connus et déjà utilisés en tant que médicaments spécifiques de cette spécialité.

La seconde substance thérapeutiquement active comprise dans la composition pharmaceutique de l'invention est donc un antibiotique, qui est déjà connu en tant que tel et déjà utilisé en tant que médicament spécifique de cette spécialité et dont l'activité est potentialisée.

Des exemples d'antibiotiques connus et déjà utilisés en tant que médicaments spécifiques de cette spécialité qui peuvent être utilisés dans la composition pharmaceutique de l'invention, et dont l'effet sera potentialisé par la première substance pure thérapeutiquement active, appartiennent à plusieurs familles : la famille des bêtalactamines représentées par l'amoxicilline et l'ampicilline, la famille des cephalosporines représentées par la céphazoline, la famille des tétracyclines représentées par la chlortétracycline, la famille des rifamycines représentées par la rifampicine, la famille des peptidiques représentés par la polymixine, la famille des aminosides représentée par la streptomycine, la famille des phénicolés représentés par le chloramphénicol, la famille des macrolides représentés par l'érythromycine.

Ces composés peuvent être utilisés seuls, ou en combinaison l'un avec l'autre.

On préfère tout particulièrement l'amoxicilline, éventuellement associée à l'acide clavulanique, l'ampicilline, la tétracycline, l'érythromycine, la streptomycine, le chloramphénicol, la rifampicine, l'isoniazide, la céphazoline, et la polymixine B utilisés en combinaison avec tout particulièrement le carvacrol, le carvéol, le thymol, l'alpha- et la bêta-ionone, comme défini dans la revendication 1.

Etant donné l'effet potentialisateur exercé par la première substance thérapeutiquement active définie dans l'invention, d'autres antibiotiques connus ou à venir pourraient également être utilisés avec succès; ce qui ne fait pas partie de l'invention revendiquée.

La composition pharmaceutique selon l'invention peut être formulée sous une forme adaptée pour une administration simultanée ou séquentielle desdites au moins première et seconde substances thérapeutiquement actives.

La forme galénique de la composition pharmaceutique de l'invention sera adaptée à son utilisation. Par exemple, elle pourra être utilisée sous la forme de solution, de suspension, de cachet ou autres. Les compositions pour administration parentérale sont généralement des solutions ou des suspensions stériles pharmaceutiquement acceptables qui peuvent éventuellement être préparées extemporanément au moment de l'emploi.

Pour la préparation de solutions ou de suspensions non aqueuses, on peut utiliser des huiles végétales naturelles telles que l'huile d'olive, l'huile de sésame ou l'huile de paraffine ou les esters organiques injectables tels que l'oléate d'éthyle. Les solutions stériles aqueuses peuvent être constituées d'une solution des substances thérapeutiquement actives dans l'eau. Les solutions aqueuses conviennent pour l'administration intraveineuse dans la mesure où le pH est convenablement ajusté et où l'isotonicité est réalisée, par exemple par ajout d'une quantité suffisante de chlorure de sodium ou de glucose.

En effet, étant donné la structure chimique des antibiotiques, et d'autre part, vu la structure chimique du carvéol, du carvacrol, du thymol, de l'alpha-ionone, de la bêta-ionone, on pense, mais sans vouloir être lié par cette théorie, que le carvéol, le carvacrol, le thymol, l'alpha-ionone et la bêta-ionone et leurs isomères et mélanges, interagissent avec les antibiotiques, pour former des complexes ayant une structure qui diffuse plus facilement dans les liquides physiologiques de l'organisme et qui diffuse plus facilement dans le cytoplasme des cellules infectées ciblées.

Mais, il a été démontré que lorsque les différents éléments de la composition pharmaceutique de l'invention sont mélangés en présence de détergents tels que le Tween ou le Triton ou de dissolvants tels que l'éthanol et le DMSO (diméthyl sulphoxide), les molécules actives de la première et de la seconde substance thérapeutiquement active s'associent avec les molécules de détergents et de dissolvants et ne forment pas de complexe de potentialisation.

Or on a découvert que le complexe de potentialisation se forme lorsqu'on utilise une suspension aqueuse d'agar, en tant que moyen de dispersion par viscosité.

Ainsi, la composition pharmaceutique de la présente invention sera de préférence préparée sans détergent et sans solvant. Par exemple, elle sera mise en suspension aqueuse rendue visqueuse par de l'agar à une concentration non gélifiante, par exemple de 1 à 5 grammes d'agar par litre de suspension.

La composition pharmaceutique de l'invention permet de traiter des infections localisées ou systémiques à germes résistants avec des doses plus faibles de chacune desdites première et seconde substances thérapeutiquement actives que les doses nécessaires au traitement des mêmes infections à germes sensibles, par l'une ou l'autre de ces mêmes dites première et seconde substances thérapeutiquement actives seules. En effet, la composition de l'invention permet d'utiliser des doses de ladite première substance thérapeutiquement active, lorsque en combinaison avec ladite seconde substance thérapeutiquement active, environ trois à dix fois inférieures à celles nécessaires lorsque ladite première substance thérapeutiquement active est utilisée seule et des doses de ladite seconde substance thérapeutiquement active, lorsque en combinaison avec ladite première substance thérapeutiquement active, de 2 à 10 fois inférieures à celles nécessaires lorsque ladite seconde substance thérapeutiquement active est utilisée seule.

Ceci a pour conséquence d'offrir un traitement qui présente les avantages suivants:
- efficacité contre les germes sensibles avec des doses très faibles,
- efficacité contre les germes résistants à un agent thérapeutique,
- efficacité contre les germes résistants à plusieurs agents thérapeutiques,
- lutte contre les phénomènes de récidive,
- lutte contre les phénomènes de sélection de germes résistants,

Dans tous ces cas, il y a une diminution remarquable des risques de toxicité et/ou d'apparition d'effets secondaires indésirables bien connus de l'homme du métier, grâce à la potentialisation qui permet l'administration de doses très faibles.

De plus, il en résulte une diminution du coût de production du traitement étant donné la faible quantité de principes actifs utilisés.

Les compositions pharmaceutiques de l'invention peuvent se présenter sous la forme de liposomes ou sous forme d'association avec des supports tels que les cyclodextrines ou les polyéthyléneglycols.

Les compositions pharmaceutiques de l'invention représentent un moyen simple et efficace pour lutter contre les problèmes liés aux agents microbiens en général qui sont essentiellement la résistance aux agents thérapeutiques et la toxicité de ceux-ci générée par l'utilisation de fortes doses.

En effet, le carvéol, le thymol, le carvacrol l'alpha et la bêta-ionone et leurs mélanges et isomères, sont des molécules simples n'ayant jamais été décrites comme ayant une toxicité quelconque et leur ajout ayant un effet potentialisateur sur la seconde substance thérapeutiquement active permet d'utiliser des doses beaucoup plus faibles de cette seconde substance thérapeutiquement active.

Le procédé de traitement des patients atteints d'une infection bactérienne consistera donc, dans une première variante, à administrer à ces patients la dose déterminée par le médecin de la composition pharmaceutique de l'invention contenant les doses appropriées de ladite au moins une première substance thérapeutiquement active, combinées aux doses appropriées de ladite au moins une seconde substance thérapeutiquement active, c'est-à-dire l'antibiotique approprié.

Dans une seconde variante, le procédé de traitement des patients atteints d'une infection bactérienne consistera à administrer à ces patients séquentiellement la dose déterminée par le médecin de ladite au moins une première substance thérapeutiquement active, puis la dose appropriée de ladite au moins une seconde substance thérapeutiquement active, c'est-à-dire l'antibiotique approprié, ou l'inverse.

A cet effet, l'invention propose une trousse contenant, au moins un premier récipient contenant une desdites premières substances thérapeutiquement actives, et au moins un second récipient contenant une desdites secondes substances thérapeutiquement actives.

Cette trousse permettra au personnel soignant de préparer à la demande soit un mélange, des doses appropriées, de(s) la première(s) substance thérapeutique voulue(s) et de(s) l'antibiotique(s) voulu(s), pour une administration simultanée, soit d'administrer séquentiellement et de façon séparée la dose appropriée de ladite au moins une première substance thérapeutiquement active, puis la dose appropriée de ladite au moins une seconde substance thérapeutiquement active, c'est-à-dire l'antibiotique approprié, ou l'inverse. Cependant, on préférera utiliser un mélange pour utilisation simultanée pour permettre au complexe de potentialisation de se former et d'agir immédiatement dès l'administration au patient.

Pour mieux faire comprendre l'invention, on va maintenant décrire à titre d'exemples purement illustratifs plusieurs modes de mise en oeuvre.

### EXEMPLE 1 : • Traitement de différentes souches de bactéries par de l'amoxicilline potentialisée par le carvéol (Amox-P)

### Test in vitro : Détermination de la concentration minimale bactéricide (CMB) sur différentes souches de bactéries.

L'expérience a été menée avec plusieurs souches de bactéries à Gram négatif et à Gram positif de sensibilités différentes isolées en milieu hospitalier. L'antibiotique utilisé est l'amoxicilline qui fait parti des antibiotiques les plus utilisés et les plus efficaces.

Des compositions pharmaceutiques antibactériennes selon l'invention ont été fabriquées en mélangeant l'amoxicilline à différentes concentrations avec le carvéol à une concentration infra inhibitrice de 0,3 grammes pour un litre de solution ou d'excipient (équivalent à 0,3mg/ml) et on a déterminé la concentration minimale d'amoxicilline en combinaison avec les 0,3 mg/ml de carvéol, pour obtenir une efficacité bactéricide du mélange. Dans chaque cas, l'activité antibiotique a été testée soit avec de l'amoxicilline seule, soit avec du carvéol seul, soit avec la composition selon l'invention.

Le tableau 1 ci-après donne les résultats de tests statiques qui mesurent la concentration minimale bactéricide (CMB).

**Tableau 1**

| Bactéries en phase de croissance exponentielle | Composition de l'invention | Composition de l'invention | Carvéol seul |
|---|---|---|---|
| | CMB (µg/ml) | CMB(µg/ml) | CMB (mg/ml) |
| *Escherichia coli* | >50 | 1 | 2 |
| *Salmonella typhimurium* | >50 | 1 | 2 |
| *Klebsiella pneumoniae* | >50 | 5 | 2 |
| *Bacillus subtilis* | >50 | 1 | 2 |
| *Staphilococcus epidermidis* | >50 | 5 | 2 |
| *Staphilococcus aureus* | >50 | 1 | 2 |

Il apparaît clairement du tableau 1 que la composition selon l'invention a une action bactéricide remarquable sur ces souches de sensibilité variable comparées à l'amoxicilline seule ou au carvéol seul.

En effet, on constate à la lecture du tableau 1 que la composition de l'invention a une action bactéricide remarquable sur les différentes souches bactériennes testées comparées à l'amoxicilline seule ou au carvéol seul, et qu'en utilisant le carvacrol à 0,3 mg/ml soit à une concentration six fois plus faible que la CMB du carvéol seul, la concentration en amoxicilline permettant d'obtenir une efficacité bactéricide est au moins dix fois inférieure à la concentration d'amoxicilline seule capable d'exercer une action bactéricide.

### Exemple 2

### Tests in vivo:

Des lots de 10 souris expérimentalement infectées par voie intrapéritonéale par 1000 000 de cellules (Unités Formant Colonie) de klebsiella pneumoniae résistante à l'amoxicilline.

Le premier lot est constitué de souris témoins infectées non traitées

Le deuxième lot est constitué de souris infectées et traitées 24h après l'infection (par gavage) par de l'amoxicilline seule à raison de 10 mg/jour par Kg de poids des animaux.

Le troisième lot est constitué de souris infectées et traitées 24h après l'infection (par gavage) par 120 mg/jour/kg de carvéol seul.

Le quatrième lot est constitué de souris infectées et traitées 24h après l'infection (par gavage) par la composition pharmaceutique (AMOX-P) selon l'invention à raison de 2 mg/kg /jour d'amoxicilline et de 120 mg/kg /jour de carvéol.

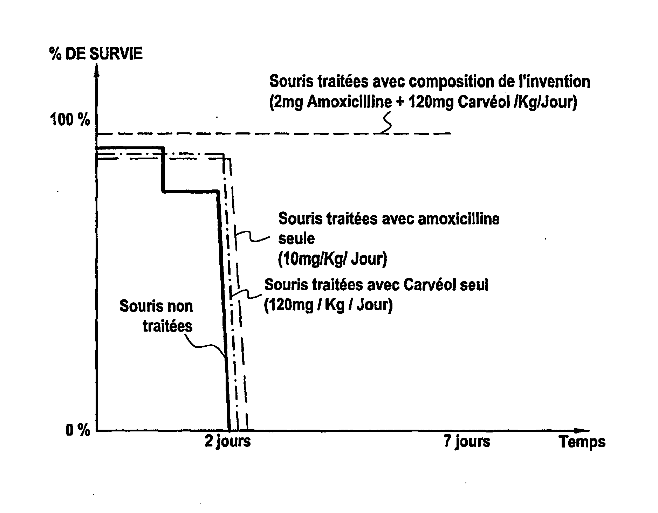

On mesure dans le temps le pourcentage de survie des souris. Les résultats de ces tests sont représentés en figure 1.

Cette figure montre les pourcentages de survie de chaque groupe de souris.

On voit que seules les souris traitées par la composition selon l'invention restent vivantes dix jours après l'infection.

Toutes les autres meurent entre le deuxième et le troisième jour après l'infection.

La recherche de *Klebsiella pneumoniae* dans les organes des animaux morts pendant l'expérience (animaux témoins non traités et animaux traités à l'amoxicilline seule) montre une forte charge de germe au niveau des poumons, du rein et de la moelle osseuse. Par contre, tous les animaux traités avec la composition pharmaceutique de l'invention, sacrifiés sept jours après l'arrêt du traitement montrent une absence du germe au niveau des poumons et de la moelle osseuse de la totalité des animaux. Au niveau du rein, seuls trois animaux sur dix portaient encore une charge très faible de *Klebsiella pneumoniae* qui est équivalente à 10 % de la charge retrouvée chez les animaux témoins infectés non traités.

Par conséquent, il apparaît clairement que la potentialisation de l'amoxicilline par sa combinaison avec le carvéol dans cet exemple permet d'obtenir des résultats surprenants quant à la diminution de la Concentration Minimale Bactéricide *in vitro* et que cette potentialisation est retrouvée *in vivo* sur un modèle d'infection systémique.

Comme l'infection systémique représente une des formes d'infection qui menacent la vie des patients et les plus difficiles à traiter surtout qu'ils présentent des problèmes de récidive avec sélection de germes de plus en plus résistants, la composition pharmaceutique de l'invention présente des avantages certains.

La méthode de traitement d'une infection bactérienne consistera à administrer à un patient atteint d'une infection bactérienne, de manière simultanée ou séquentielle la dose déterminée par le médecin d'au moins une première substance pure thérapeutiquement active choisie parmi le carvéol, le thymol, l'eugénol, le bornéol, le carvacrol, l'alpha-ionone, la bêta-ionone et les isomères, dérivés et mélanges de ceux-ci, et la dose déterminée d'au moins une seconde substance thérapeutiquement active qui est un antibiotique bien connu et déjà utilisé cliniquement en tant que médicament spécifique de cette spécialité.

Généralement, on administrera de manière simultanée ou séquentielle, à un patient atteint d'une infection due à une bactérie entre 10 et 200 mg/kg de poids du patient/jour de ladite première substance thérapeutiquement active, et entre 1 et 100 mg/kg de poids du patient/jour de ladite seconde substance thérapeutiquement active qui est un antibiotique connu et déjà utilisé en tant que médicament spécifique de cette spécialité.

### EXEMPLE 3 : Traitement de différentes souches de bactéries par de l'ampicilline potentialisée par le carvéol (Ampi-P)

L'expérience a été menée avec plusieurs souches de bactéries résistantes isolées en milieu hospitalier. L'agent antibiotique est l'ampicilline qui est de la famille des bêtalactamines et qui fait partie des agent antibiotiques les plus utilisés. Une composition pharmaceutique antibiotique selon l'invention a été fabriquée en mélangeant l'ampicilline à différentes concentrations avec le carvéol à une concentration infrainhibitrice de 0,3 g pour un litre de solution ou d'excipient. Cette composition pharmaceutique de l'invention est notée Ampi-P, pour Ampicilline potentialisée. Dans chaque cas, l'activité antibiotique a été testée soit avec de l'ampicilline seule, soit avec du carvéol seul, soit avec la composition selon l'invention.

Le tableau 2 ci-après donne les résultats de tests statiques qui mesurent la concentration minimale inhibitrice (CMI) et la concentration minimale bactéricide (CMB) en µg/ml.

**Tableau 2**

| Bactéries en phase de croissance exponentielle | Ampicilline seule | Ampi-P | Carvéol seul |
|---|---|---|---|
| | CMI (µg/ml) | CMB (µg/ml) | CMB (µg/ml) |
| *Escherichia*. *coli* | >50 | 5 | 2000 |
| *Salmonella. tiphimurium* | >50 | 1 | 2000 |
| *Bacillus. subtilis* | >50 | 10 | 2000 |
| *Staphylococcus epidermidis* | >50 | 5 | 2000 |

Il apparaît du tableau 2 que la composition selon l'invention a une action bactéricide remarquable sur les souches de bactéries testées en comparaison à l'ampicilline seule ou au carvéol seul.

En effet, on constate à la lecture du tableau 3 qu'en utilisant le carvéol à 0,3 mg/ml soit à une concentration 6,6 fois plus faible que la CMB du carvéol seul, la concentration en ampicilline permettant d'obtenir une efficacité bactéricide est de cinq à cinquante fois inférieure à la concentration d'ampicilline seule capable d'exercer une action bactériostatique.

Ainsi, on constate que la potentialisation de l'ampicilline par le carvéol permet non seulement de réduire considérablement la dose d'ampicilline mais aussi d'obtenir une action bactéricide à faible dose.

### EXEMPLE 4: Traitement de différentes souches de bactéries par une céphalosporine potentialisée par l'alpha-ionone et la bêta-ionone (Cépha-P)

L'expérience a été menée avec plusieurs souches de bactéries résistantes isolées en milieu hospitalier. L'agent antibiotique est la céphazoline qui est de la famille des céphalosporines, une autre classe de bêtalactamines et qui fait partie des agents antibiotiques les plus utilisés. Une composition pharmaceutique antibiotique selon l'invention a été fabriquée en mélangeant la céphazoline à différentes concentrations avec l'alpha-ionone ou la bêta-ionone à une concentration infrainhibitrice de 0,3 g pour un litre de solution ou d'excipient. Cette composition pharmaceutique de l'invention est notée Cépha-P, pour céphazoline potentialisée. Dans chaque cas, l'activité antibiotique a été testée soit avec de la céphazoline seule, soit avec de l'alpha-ionone ou la bêta-ionone seules, soit avec la composition selon l'invention.

Le tableau 3 ci-après donne les résultats de tests statiques qui mesurent la concentration minimale inhibitrice (CMI) et la concentration minimale bactéricide (CMB) en µg/ml.

**Tableau 3**

| Bactéries en phase de croissance exponentielle | Céphazoline seule | Cépha-P alpha-ionone | Cépha-P bêta-ionone | Alpha-ionone seule | Bêta-ionone seule |
|---|---|---|---|---|---|
| | CMI (µg/ml) | CMB (µg/ml) | CMB (µg/ml) | CMB (µg/ml) | CMB (µg/ml) |
| *Escherichia. coli* | >50 | 5 | 5 | 2000 | 2000 |
| *Salmonella. tiphimurium* | >50 | 5 | 5 | 2000 | 2000 |
| *Bacillus. subtilis* | >50 | 5 | 5 | 2000 | 2000 |
| *Staphylococcus epidermidis* | > 50 | 5 | 5 | 2000 | 2000 |

Il apparaît du tableau 3 que les compositions selon l'invention ont une action bactéricide remarquable sur les souches de bactéries testées en comparaison à la céphazoline seule ou à l'alpha-ionone seule ou à la bêta-ionone seule.

En effet, on constate à la lecture du tableau 3 qu'en utilisant l'alpha ou la bêta-ionone à 0,3 mg/ml soit à une concentration 6,6 fois plus faible que la CMB de l'alpha- ou de la bêta-ionone seules, la concentration en céphazoline permettant d'obtenir une efficacité bactéricide est au moins dix fois inférieure à la concentration de céphazoline seule capable d'exercer une action bactériostatique.

Ainsi, on constate que la potentialisation de la céphazoline par l'alpha- ou la bêta-ionone permet non seulement de réduire considérablement la dose de céphazoline mais aussi d'obtenir une action bactéricide à faible dose.

### EXEMPLE 5: Traitement de différentes souches de bactéries par la polymixine B potentialisée par le carvéol (plymix-P)

L'expérience a été menée avec plusieurs souches de bactéries résistantes isolées en milieu hospitalier. L'agent antibiotique est la polymixine B qui est de la famille des peptides et qui fait partie des agent antibiotiques les plus anciens. Une composition pharmaceutique antibiotique selon l'invention a été fabriquée en mélangeant la polymixine B à différentes concentrations avec le carvéol à une concentration infrainhibitrice de 0,3 g pour un litre de solution ou d'excipient. Cette composition pharmaceutique de l'invention est notée polymix-P, pour polymixine B potentialisée. Dans chaque cas, l'activité antibiotique a été testée soit avec de la polymixine B seule, soit avec du carvéol seul, soit avec la composition selon l'invention.

Le tableau 4 ci-après donne les résultats de tests statiques qui mesurent la concentration minimale inhibitrice (CMI) et la concentration minimale bactéricide (CMB) en µg/ml.

**Tableau 4**

| Bactéries en phase de croissance exponentielle | polymixine B seule | Polymix-P | Carvéol seul |
|---|---|---|---|
| | CMI (µg/ml) | CMB (µg/ml) | CMB (µg/ml) |
| *Echerichia. coli* | > 50 | 10 | 2000 |
| *Salmonella. tiphimurium* | 25 | 10 | 2000 |
| *Bacillus subtilis* | > 50 | 5 | 2000 |
| *Staphylococcus epidermidis* | >50 | 5 | 2000 |

Il apparaît du tableau 4 que la composition selon l'invention a une action bactéricide remarquable sur les souches de bactéries testées en comparaison à la polymixine B seule ou au carvéol seul.

En effet, on constate à la lecture du tableau 4 qu'en utilisant le carvéol à 0,3 mg/ml soit à une concentration 6,6 fois plus faible que la CMB du carvéol seul, la concentration en polymixine B permettant d'obtenir une efficacité bactéricide est 2,5 à dix fois inférieure à la concentration de polymixine B seule capable d'exercer une action bactéricide.

Ainsi, on constate que la potentialisation de la polymixine B par le carvéol permet non seulement de réduire considérablement la dose de polymixine B pour les bactéries résistantes à Gram négatif (*E. coli et S. typhymurium*) mais aussi d'élargir son spectre d'activité à des bactéries à Gram positif *(S. epidermidis, B. subtilis)* normalement insensibles à la polymixine B.

### EXEMPLE 6: Traitement de différentes souches de bactéries par le chloramphénicol potentialisé par le carvéol (Chloram-P)

L'expérience a été menée avec plusieurs souches de bactéries résistantes isolées en milieu hospitalier. L'agent antibiotique est le chloramphénicol qui est de la famille des phénicolés et qui fait partie des agents antibiotiques les plus anciens. Une composition pharmaceutique antibiotique selon l'invention a été fabriquée en mélangeant le chloramphénicol à différentes concentrations avec le carvéol à une concentration infrainhibitrice de 0,3 g pour un litre de solution ou d'excipient. Cette composition pharmaceutique de l'invention est notée Chloram-P, pour chloramphénicol potentialisé. Dans chaque cas, l'activité antibiotique a été testée soit avec du chloramphénicol seul, soit avec du carvéol seul, soit avec la composition selon l'invention.

Le tableau 5 ci-après donne les résultats de tests statiques qui mesurent la concentration minimale inhibitrice (CMI) et la concentration minimale bactéricide (CMB) en µg/ml.

**Tableau 5**

| Bactéries en phase de croissance exponentielle | Chloramphénicol seul | Chloram-P | Carvéol seul |
|---|---|---|---|
| | CMI (µg/ml) | CMB (µg/ml) | CMB (µg/ml) |
| *Escherichia. coli* | > 50 | 2 | 2000 |
| *Salmonella, tiphimurium* | > 50 | 1 | 2000 |
| *Bacillus. subtilis* | > 50 | 5 | 2000 |
| *Staphy*/*ococcus epidermidis* | > 50 | 5 | 2000 |

Il apparaît du tableau 5 que la composition selon l'invention a une action bactéricide remarquable sur les souches de bactéries testées en comparaison au chloramphénicol seul ou au carvéol seul.

En effet, on constate à la lecture du tableau 5 qu'en utilisant le carvéol à 0,3 mg/ml soit à une concentration 6,6 fois plus faible que la CMB du carvéol seul, la concentration en chloramphénicol permettant d'obtenir une efficacité bactéricide est dix à cinquante fois inférieure à la concentration de chloramphénicol seul capable d'exercer une action bactéricide.

Ainsi, on constate que la potentialisation du chloramphénicol par le carvéol permet non seulement de réduire considérablement la dose de chloramphénicol pour les bactéries résistantes à Gram négatif (*E. coli et S. typhymurium)* mais aussi d'exercer une action bactéricide sur des bactéries à Gram positif (*S. epidermidis, B. subtilis)* sur lesquelles l'action du chloramphénicol est normalement seulement bactériostatique.

### EXEMPLE 7: Traitement de différentes souches de bactéries par la Chlortétracycline potentialisée par le carvéol (Tetra-P)

L'expérience a été menée avec plusieurs souches de bactéries résistantes isolées en milieu hospitalier. L'agent antibiotique est la chlortétracycline qui fait partie des agents antibiotiques les plus anciens. Une composition pharmaceutique antibiotique selon l'invention a été fabriquée en mélangeant la chlortétracycline à différentes concentrations avec le carvéol à une concentration infrainhibitrice de 0,3 g pour un litre de solution ou d'excipient. Cette composition pharmaceutique de l'invention est notée Tetra-P, pour chlortétracycline potentialisée. Dans chaque cas, l'activité antibiotique a été testée soit avec de la chlortétracycline seule, soit avec du carvéol seul, soit avec la composition selon l'invention.

Le tableau 6 ci-après donne les résultats de tests statiques qui mesurent la concentration minimale inhibitrice (CMI) et la concentration minimale bactéricide (CMB) en µg/ml.

**Tableau 6**

| Bactéries en phase de croissance exponentielle | Chlortétracycline seule | Tetra-P | Carvéol seul |
|---|---|---|---|
| | CMI (µg/ml) | CMB (µg/ml) | CMB (µg/ml) |
| *Escherichia. coli* | > 50 | 2 | 2000 |
| *Salmonella*. *tiphimurium* | > 50 | 1 | 2000 |
| *Bacillus. subtilis* | > 50 | 2 | 2000 |
| *Staphylococcus aureus* | > 50 | 2 | 2000 |

Il apparaît du tableau 6 que la composition selon l'invention a une action bactéricide remarquable sur les souches de bactéries testées en comparaison à la chlortétracycline seule ou au carvéol seul.

En effet, on constate à la lecture du tableau 6 qu'en utilisant le carvéol à 0,3 mg/ml soit à une concentration 6,6 fois plus faible que la CMB du carvéol seul, la concentration en chlortétracycline permettant d'obtenir une efficacité bactéricide est vingt cinq à cinquante fois inférieure à la concentration de chlortétracycline seule capable d'exercer une action bactéricide.

Ainsi, on constate que la potentialisation de la tétracycline par le carvéol permet non seulement de réduire considérablement la dose de chlortétracycline mais aussi d'exercer une action bactéricide à très faible dose.

### EXEMPLE 8: Traitement de différentes souches de bactéries par la Streptomycine potentialisée par le carvéol (Strept-P)

L'expérience a été menée avec plusieurs souches de bactéries résistantes isolées en milieu hospitalier. L'agent antibiotique est la streptomycine membre important de la famille des aminosides qui fait partie des agents antibiotiques les plus importants. Une composition pharmaceutique antibiotique selon l'invention a été fabriquée en mélangeant la streptomycine à différentes concentrations avec le carvéol à une concentration infrainhibitrice de 0,3 g pour un litre de solution ou d'excipient. Cette composition pharmaceutique de l'invention est notée Strepto-P, pour streptomycine potentialisée. Dans chaque cas, l'activité antibiotique a été testée soit avec de la streptomycine seule, soit avec du carvéol seul, soit avec la composition selon l'invention.

Le tableau 7 ci-après donne les résultats de tests statiques qui mesurent la concentration minimale inhibitrice (CMI) et la concentration minimale bactéricide (CMB) en µg/ml.

**Tableau 7**

| Bactéries en phase de croissance exponentielle | Streptomycine seule | Strepto-P | Carvéol seul |
|---|---|---|---|
| | CMI (µg/ml) | CMB (µg/ml) | CMB (µg/ml) |
| *Escherichia. coli* | > 50 | 5 | 2000 |
| *Salmonella. tiphimurium* | > 50 | 5 | 2000 |
| *Bacillus*. *subtilis* | > 50 | 5 | 2000 |
| *Staphylococcus aureus* | > 50 | 5 | 2000 |

Il apparaît du tableau 7 que la composition selon l'invention a une action bactéricide remarquable sur les souches de bactéries testées en comparaison à la streptomycine seule ou au carvéol seul.

En effet, on constate à la lecture du tableau 7 qu'en utilisant le carvéol à 0,3 mg/ml soit à une concentration 6,6 fois plus faible que la CMB du carvéol seul, la concentration en streptomycine permettant d'obtenir une efficacité bactéricide est au moins dix fois inférieure à la concentration de streptomycine seule capable d'exercer une action bactéricide.

Ainsi, on constate que la potentialisation de la streptomycine par le carvéol permet non seulement de réduire considérablement la dose de streptomycine mais aussi d'exercer une action bactéricide à très faible dose.

### EXEMPLE 9: Traitement de différentes souches de bactéries par l'éryrthromyrcine potentialisée par le carvéol (Erythro-P)

L'expérience a été menée avec plusieurs souches de bactéries résistantes isolées en milieu hospitalier. L'agent antibiotique est l'érythromycine membre important de la famille des macrolides qui fait partie des agents antibiotiques les plus importants. Une composition pharmaceutique antibiotique selon l'invention a été fabriquée en mélangeant l'érythromycine à différentes concentrations avec le carvéol à une concentration infrainhibitrice de 0,3 g pour un litre de solution ou d'excipient. Cette composition pharmaceutique de l'invention est notée Erythro-P, pour érythromycine potentialisée. Dans chaque cas, l'activité antibiotique a été testée soit avec de l'érythromycine seule, soit avec du carvéol seul, soit avec la composition selon l'invention.

Le tableau 8 ci-après donne les résultats de tests statiques qui mesurent la concentration minimale inhibitrice (CMI) et la concentration minimale bactéricide (CMB) en µg/ml.

**Tableau 8**

| Bactéries en phase de croissance exponentielle | l'érythromycine seule | Erythro-P | Carvéol seul |
|---|---|---|---|
| | CMI (µg/ml) | CMB (µg/ml) | CMB (µg/ml) |
| *Escherichia. coli* | > 50 | 10 | 2000 |
| *Salmonella. tiphimurium* | > 50 | 10 | 2000 |
| *Bacillus*. *subtilis* | > 50 | 15 | 2000 |
| *Staphylococcus aureus^{.}* | > 50 | 25 | 2000 |

Il apparaît du tableau 8 que la composition selon l'invention a une action bactéricide remarquable sur les souches de bactéries testées en comparaison à l'érythromycine seule ou au carvéol seul.

En effet, on constate à la lecture du tableau 8 qu'en utilisant le carvéol à 0,3 mg/ml soit à une concentration 6,6 fois plus faible que la CMB du carvéol seul, la concentration en érythromycine permettant d'obtenir une efficacité bactéricide est au moins 2 à cinq fois inférieure à la concentration d'érythromycine seule capable d'exercer une action bactéricide.

Ainsi, on constate que la potentialisation de l'érythromycine par le carvéol permet de réduire considérablement la dose d'érythromycine capable d'exercer une action bactéricide.

### EXEMPLE 10: Traitement de différentes souches de mycobactéries par la rifampicine potentialisée par le carvacrol (Rifam-P)

L'expérience a été menée avec deux souches de mycobactéries résistantes isolées en milieu vétérinaire. L'agent antibiotique est la rifampicine membre important de la famille des antituberculeux. Une composition pharmaceutique antibiotique selon l'invention a été fabriquée en mélangeant la rifampicine à différentes concentrations avec le carvacrol à une concentration infrainhibitrice de 0,3 g pour un litre de solution ou d'excipient. Cette composition pharmaceutique de l'invention est notée Rifam-P, pour rifampicine potentialisée. Dans chaque cas, l'activité antibiotique a été testée soit avec de la rifampicine seule, soit avec du carvacrol seul, soit avec la composition selon l'invention.

Le tableau 9 ci-après donne les résultats de tests statiques qui mesurent la concentration minimale inhibitrice (CMI) et la concentration minimale bactéricide (CMB) en µg/ml.

**Tableau 9**

| Bactéries en phase de croissance exponentielle | Rifampicine seule | Rifam-P | Carvacrol seul |
|---|---|---|---|
| | CMI (µg/ml) | CMB (µg/ml) | CMB (µg/ml) |
| *Mycobacterium fleii* | > 75 | 2,5 | 1000 |
| *Mycobacterium fortuitum* | > 75 | 5 | 1000 |

Il apparaît du tableau 9 que la composition selon l'invention a une action bactéricide remarquable sur les souches de mycobactéries testées en comparaison à la rifampicine seule ou au carvacrol seul.

En effet, on constate à la lecture du tableau 9 qu'en utilisant le carvacrol à 0,3 mg/ml soit à une concentration 3,3 fois plus faible que la CMB du carvacrol seul, la concentration en rifampicine permettant d'obtenir une efficacité bactéricide est au moins 25 fois inférieure à la concentration de rifampicine seule capable d'exercer une action bactéricide.

Ainsi, on constate que la potentialisation de la rifampicine par le carvacrol permet de réduire considérablement la dose de rifampicine capable d'exercer une action bactéricide sur des mycobactéries à croissance rapide normalement insensible à la rifampicine.

### EXEMPLE 11: Traitement de différentes souches de mycobactéries par l'isoniazide potentialisé par le thymol (Izon-P)

L'expérience a été menée avec deux souches de mycobactéries résistantes isolées en milieu vétérinaire. L'agent antibiotique est l'isoniazide membre important de la famille des antituberculeux. Une composition pharmaceutique antibiotique selon l'invention a été fabriquée en mélangeant l'isoniazide à différentes concentrations avec le thymol à une concentration infrainhibitrice de 0,3 g pour un litre de solution ou d'excipient. Cette composition pharmaceutique de l'invention est notée Izon-P, pour isoniazide potentialisé. Dans chaque cas, l'activité antibiotique a été testée soit avec de l'isoniazide seul, soit avec du thymol seul, soit avec la composition selon l'invention.

Le tableau 10 ci-après donne les résultats de tests statiques qui mesurent la concentration minimale inhibitrice (CMI) et la concentration minimale bactéricide (CMB) en µg/ml.

**Tableau 10**

| Bactéries en phase de croissance exponentielle | isoniazide seul | Izon-P | Carvacrol seul |
|---|---|---|---|
| | CMI (µg/ml) | CMB (µg/ml) | CMB (µg/ml) |
| *Mycobacterium fleii* | > 50 | 1 | 1000 |
| *Mycobacterium fortuitum* | > 50 | 2 | 1000 |

Il apparaît du tableau 10 que la composition selon l'invention a une action bactéricide remarquable sur les souches de mycobactéries testées en comparaison à l'isoniazide seul ou au thymol seul.

En effet, on constate à la lecture du tableau 10 qu'en utilisant le thymol à 0,3 mg/ml soit à une concentration 3,3 fois plus faible que la CMB du thymol seul, la concentration en isoniazide permettant d'obtenir une efficacité bactéricide est au moins 25 fois inférieure à la concentration d'isoniazide seul capable d'exercer une action bactéricide.

Ainsi, on constate que la potentialisation de l'isoniazide par le thymol permet de réduire considérablement la dose d'isoniazide capable d'exercer une action bactéricide sur des mycobactéries à croissance rapide normalement insensibles à l'isoniazide.

### Exemple 12: Sélection de mutants résistants en présence d'amoxicilline potentialisée par le carvacrol :

Dans le but de vérifier que la potentialisation selon l'invention permet d'empêcher la sélection de mutants résistants, nous avons procédé à l'expérience suivante :
Des souches d'*Escherichia. coli* sensibles à l'amoxicilline à la concentration de 5 µg/ml sont cultivées à une concentration infrainhibitrice de 3µg/ml ensuite repiquées dans des milieux nutritifs (Muller Hinton) contenant des concentrations de plus en plus grandes d'amoxicilline (4 puis 5 puis 6 µg/ml ...). La même opération est effectuée avec l'amoxicilline potentialisée par du carvacrol à une concentration deux fois plus faible que la CMI du carvacrol seul soit 500µg/ml. Le principe de cette expérience est qu'à chaque repiquage, un mutant résistant à la nouvelle concentration d'amoxicilline va se multiplier pour constituer une culture de souche plus résistante que la culture à partir de laquelle le repiquage a été réalisé.

Le tableau 11 ci-après donne les résultats de cette expérience qui montre la sélection de mutants de plus en plus résistants et le nombre de repiquage nécessaire pour les obtenir.

**Tableau 11**

| | Concentration initiale (µg/ml) | Nombre de repiquages | Concentration médiane (µg/ml) | Nombre de repiquages | Concentration finale (µg/ml) |
|---|---|---|---|---|---|
| Amoxicilline seule | 3 | 4 | 17 | 9 | 50 |
| Amoxicililne potentialisée | 3 | 14 | 17 | / | / |

Il apparaît du tableau 11 que la composition selon l'invention nécessite 14 repiquages progressifs pour sélectionner un mutant résistant à la concentration de 17 µg/ml d'amox-P en partant de 3µg/ml, alors qu'avec l'amoxicilline seule, des mutants résistants à 17µg/ml ont été obtenus en 4 repiquages seulement. Des mutants encore plus résistants allant jusqu'à 50 µg/ml d'amoxicilline seule ont été obtenus en 9 repiquages. Avec l'amox-P, aucun mutant résistant à une concentration supérieure à 17µg/ml n'a pu être sélectionné.

En effet, on constate à la lecture de ces résultats d'une part, qu'en utilisant le carvacrol à 0,5 mg/ml soit à une concentration deux fois plus faible que la CMI du carvacrol seul il est beaucoup plus difficile de sélectionner des mutants résistants (14 repiquages) à la composition de l'invention comparativement à l'amoxicilline seule (4 repiquages). D'autre part, la sélection de mutants résistant à l'amoxicilline seule continue de plus en plus facilement jusqu'à 50 µg/ml, concentration à laquelle l'expérience a été arrêtée, alors qu'en présence de la composition de l'invention, la sélection de mutants résistant a été plafonné à 17µg/ml.

Ainsi, on constate que la potentialisation de l'amoxicilline par le carvacrol permet de réduire considérablement la possibilité de sélectionner des mutants résistants.

### Exemple 13 : Sélection de mutants résistants en présence de l'association amoxicilline et acide clavulanique potentialisée par le carvacrol :

L'association est réalisée aux proportions 1 gramme d'amoxicilline avec 0,125 gramme d'acide clavulanique.

Dans le but de vérifier que la potentialisation selon l'invention permet d'empêcher la sélection de mutants résistants, nous avons procédé à l'expérience suivante :
Des souches d'*Escherichia, coli* sensibles à l'association amoxicilline et acide clavulanique à la concentration de 5 µg/ml (d'amoxicilline) sont cultivées à une concentration infrainhibitrice de 3µg/ml ensuite repiquées dans des milieux nutritifs (Muller Hinton) contenant des concentrations de plus en plus grandes de l'association amoxicilline et acide clavulanique (4 puis 5 puis 6 µg/ml ...). La même opération est effectuée avec l'association amoxicilline et acide clavulanique potentialisée par du carvacrol à une concentration deux fois plus faible que la CMI du carvacrol seul soit 500µg/ml. Le principe de cette expérience est qu'a chaque repiquage, un mutant résistant à la nouvelle concentration de l'association amoxicilline et acide clavulanique va se multiplier pour constituer une culture de souche plus résistante que la culture à partir de laquelle le repiquage a été réalisé.

Le tableau 12 ci-après donne les résultats de cette expérience qui montre la sélection de mutants de plus en plus résistants et le nombre de repiquages nécessaires pour les obtenir.

**Tableau 12**

| | Concentration initiale (µg/ml) | Nombre de repiquages | Concentration médiane (µg/ml) | Nombre de repiquages | Concentration finale (µg/ml) |
|---|---|---|---|---|---|
| L'association amoxicilline et acide clavulanique seule | 3 | 8 | 20 | 13 | 50 |
| L'association amoxicilline et acide davulanique potentialisée | 3 | 17 | 20 | / | / |

Il apparaît du tableau 12 que la composition selon l'invention nécessite 17 repiquages progressifs pour sélectionner un mutant résistant à la concentration de 20 µg/ml en partant de 3 µg/ml, alors qu'avec l'association amoxicilline et acide clavulanique seule, des mutants résistants à 20 µg/ml ont été obtenus en 8 repiquages seulement. Des mutants encore plus résistants allant jusqu'à 50 µg/ml de l'association amoxicilline et acide clavulanique seule ont été obtenus en 13 repiquages. Avec la composition de l'invention, aucun mutant résistant à une concentration supérieure à 20 µg/ml n'a pu être sélectionné.

En effet, on constate à la lecture de ces résultats d'une part, qu'en utilisant le carvacrol à 0,5 mg/ml soit à une concentration deux fois plus faible que la CMI du carvacrol seul Il est beaucoup plus difficile de sélectionner des mutants résistants (17 repiquages) à la composition de l'invention comparativement à l'association amoxicilline et acide clavulanique seule (8 repiquages). D'autre part, la sélection de mutants résistant à l'association amoxicilline et acide clavulanique seule continue de plus en plus facilement jusqu'à 50µg/ml à laquelle nous avons choisi d'arrêter l'expérience, alors qu'en présence de la composition de l'invention, la sélection de mutants résistant a été plafonné à 20 µg/ml.

Ainsi, on constate que la potentialisation de l'association amoxicilline et acide clavulanique par le carvacrol permet de réduire considérablement la possibilité de sélectionner des mutants résistants.

Il apparaît clairement des tous les exemples ci-dessus, que la potentialisation des antibiotiques par les dites premières substances thérapeutiquement actives permet de diminuer les doses nécessaires pour combattre les bactéries résistantes, d'élargir le spectre d'activité des antibiotiques, de transformer l'action bactériostatique en action bactéricide et de rendre beaucoup plus difficile la possibilité de sélectionner des mutants résistants.

## Revendications

1. Composition pharmaceutique **caractérisée en ce qu'**elle comprend :
- au moins une première substance thérapeutiquement active, et
- au moins une seconde substance thérapeutiquement active qui est un antibiotique,
dans laquelle
ladite première substance thérapeutiquement active est le carvéol et l'antibiotique est choisi parmi l'ampicilline, le chloramphénicol, la tétracycline, la streptomycine, l'érythromycine, la polymixine B et l'amoxicilline ; ou
ladite première substance thérapeutiquement active est le carvacrol et l'antibiotique est choisi parmi l'amoxicilline, l'amoxicilline associée à l'acide clavulanique et la rifampicine ; ou
ladite première substance thérapeutiquement active est l'alpha-ionone ou la bêta-ionone et l'antibiotique est la céphazoline.

2. Composition selon la revendication 1, **caractérisée en ce que** ladite première substance thérapeutiquement active est le carvéol et l'antibiotique est l'amoxicilline.

3. Composition selon la revendication 1 ou 2, **caractérisée en ce que** lesdites première et seconde substances thérapeutiquement actives sont mises en suspension dans une solution aqueuse d'agar.

4. Composition selon l'une quelconque des revendications 1 à 3, **caractérisée en ce qu'**elle ne contient pas de détergent ou de solvant.

5. Trousse **caractérisée en ce qu'**elle contient :
- au moins un premier récipient contenant une première substance thérapeutiquement active, et
- au moins un second récipient contenant une seconde substance thérapeutiquement active qui est un antibiotique,
dans laquelle
ladite première substance thérapeutiquement active est le carvéol et l'antibiotique est choisi parmi l'ampicilline, le chloramphénicol, la tétracycline, la streptomycine, l'érythromycine, la polymixine B et l'amoxicilline ; ou
ladite première substance thérapeutiquement active est le cravacrol et l'antibiotique est choisi parmi l'amoxicilline, l'amoxicilline associée à l'acide clavulanique et la rifampicine ; ou
ladite première substance thérapeutiquement active est l'alpha-ionone ou la bêta-ionone et l'antibiotique est la céphazoline.

6. Trousse selon la revendication 5, **caractérisée en ce que** ladite première substance thérapeutiquement active est le carvéol et l'antibiotique est l'amoxicilline.

7. Composition selon l'une quelconque des revendications 1 à 4 ou trousse selon l'une quelconque des revendications 5 à 6 pour une utilisation dans le traitement d'une infection due à une bactérie chez un patient.

8. Composition ou trousse selon la revendication 7, **caractérisée en ce que** les doses à administrer sont :
- entre 10 et 200 mg/kg de poids du patient/jour pour la première substance thérapeutiquement active, et
- entre 2 et 100 mg/kg de poids du patient/jour pour la seconde substance thérapeutiquement active de ladite composition.

## Patentansprüche

1. Pharmazeutische Zusammensetzung, **dadurch gekennzeichnet, dass** sie umfasst:
- wenigstens eine erste therapeutisch aktive Substanz, und
- wenigstens eine zweite therapeutisch aktive Substanz, die ein Antibiotikum ist,
wobei
besagte erste therapeutisch aktive Substanz Carveol ist und das Antibiotikum aus Ampicillin, Chloramphenicol, Tetracyclin, Streptomycin, Erythromycin, Polymixin B und Amoxicillin ausgewählt ist; oder
besagte erste therapeutisch aktive Substanz Cravacrol ist und das Antibiotikum aus Amoxicillin, Amoxicillin in Kombination mit Clavulansäure und Rifampicin ausgewählt ist; oder
besagte erste therapeutisch aktive Substanz alpha-Ionon oder beta-Ionon ist und das Antibiotikum Cephazolin ist; oder
besagte erste therapeutisch aktive Substanz Thymol ist und das Antibiotikum Isoniazid ist.

2. Zusammensetzung gemäß Anspruch 1, **dadurch gekennzeichnet, dass** besagte erste therapeutisch aktive Substanz Carveol ist und das Antibiotikum Amoxicillin ist.

3. Zusammensetzung gemäß Anspruch 1 oder 2, **dadurch gekennzeichnet, dass** besagte erste und zweite therapeutisch aktive Substanz in einer wässrigen Agarlösung suspendiert sind.

4. Zusammensetzung gemäß einem der Ansprüche 1 bis 3, **dadurch gekennzeichnet, dass** sie kein Detergens oder Lösemittel enthält.

5. Kit, **dadurch gekennzeichnet, dass** er enthält:
- wenigstens einen ersten Behälter, enthaltend eine erste therapeutisch aktive Substanz, und
- wenigstens einen zweiten Behälter, enthaltend eine zweite therapeutisch aktive Substanz, die ein Antibiotikum ist,
wobei
besagte erste therapeutisch aktive Substanz Carveol ist und das Antibiotikum aus Ampicillin, Chloramphenicol, Tetracyclin, Streptomycin, Erythromycin, Polymixin B und Amoxicillin ausgewählt ist; oder
besagte erste therapeutisch aktive Substanz Cravacrol ist und das Antibiotikum aus Amoxicillin, Amoxicillin in Kombination mit Clavulansäure und Rifampicin ausgewählt ist; oder
besagte erste therapeutisch aktive Substanz alpha-Ionon oder beta-Ionon ist und das Antibiotikum Cephazolin ist; oder
besagte erste therapeutisch aktive Substanz Thymol ist und das Antibiotikum Isoniazid ist.

6. Kit gemäß Anspruch 5, **dadurch gekennzeichnet, dass** besagte erste therapeutisch aktive Substanz Carveol ist und das Antibiotikum Amoxicillin ist.

7. Zusammensetzung gemäß einem der Ansprüche 1 bis 4 oder Kit gemäß einem der Ansprüche 5 bis 6 zur Verwendung in der Behandlung einer bakteriellen Infektion bei einem Patienten.

8. Zusammensetzung oder Kit gemäß Anspruch 7, **dadurch gekennzeichnet, dass** die zu verabreichenden Dosen:
- zwischen 10 und 200 mg/kg Körpergewicht des Patienten/Tag für die erste therapeutisch aktive Substanz, und
- zwischen 2 und 100 mg/kg Körpergewicht des Patienten/Tag für die zweite therapeutisch aktive Substanz besagter Zusammensetzung betragen.

## Claims

1. A pharmaceutical composition **characterized in that** it comprises:
- at least one first therapeutically active substance, and
- at least one second therapeutically active substance which is an antibiotic,
wherein
said first therapeutically active substance is carveol and the antibiotic is selected from the group consisting of ampicillin, chloramphenicol, tetracycline, streptomycin, erythromycin, polymyxin B and amoxicillin; or
said first therapeutically active substance is carvacrol and the antibiotic is selected from the group consisting of amoxicillin, amoxicillin in association with clavulanic acid, and rifampicin; or
said first therapeutically active substance is alpha-ionone or beta-ionone and the antibiotic is cephazolin.

2. Composition according to claim 1, **characterized in that** said first therapeutically active substance is carveol and the antibiotic is amoxicillin.

3. Composition according to claim 1 or 2, **characterized in that** said first and second therapeutically active substances are suspended in an aqueous agar solution.

4. Composition according to any one of claims 1 to 3, **characterized in that** said composition does not include any detergent or solvent

5. Kit **characterized in that** it comprises:
- at least one first container containing a first therapeutically active substance, and
- at least one second container containing a second therapeutically active substance which is an antibiotic,
wherein
said first therapeutically active substance is carveol and the antibiotic is selected from the group consisting of ampicillin, chloramphenicol, tetracycline, streptomycin, erythromycin, polymyxin B and amoxicillin; or
said first therapeutically active substance is carvacrol and the antibiotic is selected from the group consisting of amoxicillin, amoxicillin in association with clavulanic acid, and rifampicin;
or
said first therapeutically active substance is alpha-ionone or beta-ionone and the antibiotic is cephazolin.

6. Kit according to claim 5, **characterized in that** said first therapeutically active substance is carveol and the antibiotic is amoxicillin.

7. Composition of any one of claims 1 to 4 or kit of any one of claims 5 to 6 for use in the treatment of an infection caused by bacteria in a patient.

8. Composition or kit of claim 7, **characterized in that** doses to be administrated are:
- between 10 and 200 mg/kg of body weight/day of the first therapeutically active substance, and
- between 2 and 100 mg/kg of body weight/day of the second therapeutically active substance of said composition.
